# EUROPEAN PATENT APPLICATION

(11) **EP 4 595 924 A1**
(43) Date of publication of application: **06.08.2025**
(21) Application number: 23871053.7
(22) Date of filing: 28.09.2023
(51) Int. Cl.: A61F 2/24, A61F 2/966

(54) **PROSTHESIS SYSTEM**

(30) Priority: 30.09.2022 CN 202222625620 U; 30.09.2022 CN 202211216019
(71) Applicant: Enlight Medical Technologies (Shanghai) Co., Ltd., Shanghai 201318 (CN)
(72) Inventor: HUANG, Zhen, Shanghai 201315 (CN); PAN, Fengyang, Shanghai 201315 (CN); GONG, Zhenpeng, Shanghai 201315 (CN); WANG, Ben, Shanghai 201315 (CN)
(74) Representative: Balder IP Law, S.L.
(86) International application number: PCT/CN2023/122740
(87) International publication number: WO 2024/067828

(57) **Abstract**

Provided is a prosthesis system. A prosthesis comprises a base (210) and a first movable member, the base (210) being provided with a base body (211), a first coupling part (212), and a first limiting part (213); and a conveying device (100), which comprises a connecting shaft (110) and a first control wire (120) which is provided with a first segment (121) and a second segment (122). The connecting shaft (110) is provided with a shaft body (111), a second coupling part (112) and a second limiting part (113). When the first coupling part (212) and the second coupling part (112) are coupled, a first limiting channel (300) in a locked state is formed between the first limiting part (213) and the second limiting part (113). The second segment (122) is constrained at the first limiting channel (300). After the first coupling part (212) is separated from the second coupling part (112), the first limiting channel (300) in an unlocked state is formed between the first limiting part (213) and the second limiting part (113). The first control wire (120) can be separated from the first limiting channel (300). According to the prosthesis system, under the condition that the state of the prosthesis can be safely and reliably adjusted, the situation that the control wire (120) is prone to knotting and clamping as in the prior art can be avoided, such that an operation risk is reduced.

## Description

### CROSS-REFERENCE TO RELATED APPLICATIONS

The present application is based on Chinese patent application No. 202222625620.7 filed on September 30, 2022 and Chinese patent application No. 202211216019.0 filed on September 30, 2022, and claims priority to these Chinese patent applications. The entire contents of these Chinese patent applications are hereby incorporated into the present application by reference.

### TECHNICAL FIELD

Embodiments of the present disclosure relate to the technical field of medical devices, and in particular to a prosthesis system.

### BACKGROUND

To reduce the harm caused to patients by traditional open surgery, minimally invasive surgery has been developed as an alternative. Currently, minimally invasive surgeries generally involve using a delivery device to deliver a prosthesis to a target position through surgical pathways such as human lumens. After reaching the target position, the prosthesis is then indirectly manipulated by controlling the control wire of the delivery device, thereby changing its state to achieve the surgical purpose.

Chinese patent application CN111789699A discloses a mechanical structure in which a prosthesis is controlled via the control wire of a delivery device, thereby causing a change in the state of the prosthesis to achieve surgical purposes. And in this patent application, a configuration of a control wire and a prosthesis is that one end of the control wire extends from a proximal end of the delivery device to a distal end of the delivery device, then passes through a hole in the prosthesis, and finally extends to the proximal end of the delivery device. This configuration increases the risk of the control wire becoming tangled or jammed, or otherwise malfunctioning.

### SUMMARY

It is an objective of the embodiments of the present disclosure to provide a prosthesis system that can avoid risks such as the control wire becoming tangled or jammed, as seen in prior art, and can safely and reliably adjust the state of the prosthesis.

To solve the above problems, some embodiments of the present disclosure provide a prosthesis system including a prosthesis and a delivery device. The prosthesis includes a base and a first movable member. The base has a base body and, a first coupling portion and a first limiting portion arranged on the base body. The delivery device includes a connecting shaft and a first control wire. The connecting shaft includes a shaft body and, a second coupling portion and a second limiting portion arranged on the shaft body. The second coupling portion is configured to detachably couple with the first coupling portion, and the second limiting portion is configured to form a first limiting channel together with the first limiting portion. The first control wire includes a first segment and a second segment connected to each other. The first segment is configured to independently drive the first movable member to move. The first and second limiting portions are configured such that, when the first and second coupling portions are coupled, the first limiting channel in a locked state is formed by the first limiting portion and the second limiting portion, and the second segment is constrained at the first limiting channel; and when the first coupling portion and the second coupling portion are separated, the first limiting channel in an unlocked state is formed by the first limiting portion and the second limiting portion, and the first control wire is detachable from the first limiting channel.

The prosthesis system provided in the embodiments of the present disclosure, enables the delivery device to connect with the prosthesis during minimally invasive surgery via the coupling between the first coupling portion of the base and the second coupling portion of the connecting shaft. The coupling also causes the first limiting portion and the second limiting portion to form the first limiting channel in a locked state, which constrains the second segment of the first control wire, preventing the first control wire from separating from the first limiting channel. As a result, the prosthesis can be delivered to the target position inside the human body through surgical pathways such as human lumens by means of the delivery device. When the prosthesis needs to be manipulated, the first limiting channel remains in the locked state to constrain the second segment of the first control wire at the first limiting channel, so that a portion of the first segment extending out of the first limiting channel can be utilized to adjust the movement of the first movable member of the prosthesis, thereby changing the state of the prosthesis to achieve implantation of the prosthesis, and further achieving the purpose of treatment and diagnosis. After implantation of the prosthesis, the delivery device needs to be disengaged from the prosthesis. This can be achieved by disengaging the first coupling portion from the second coupling portion, so that the first limiting channel is in the unlocked state, and the second segment is no longer constrained by the first limiting channel. As a result, the first control wire can be separated from the first limiting channel, allowing it to be separated from the prosthesis. Furthermore, the base of the prosthesis and the connecting shaft of the delivery device can be detached, so as to detach the delivery device from the prosthesis, thereby facilitating the withdrawal of the delivery device from the human body to complete the implantation surgery. As such, during minimally invasive surgery, when it is required to change the state of the prosthesis, the first movable member within the prosthesis can be safely and reliably manipulated through the first control wire of the delivery device, thus changing the state of the prosthesis.

In the embodiments of the present disclosure, when configuring the first control wire and the prosthesis, it is only required to extend the control wire from the proximal end of the delivery device to the distal end of the delivery device and into the first limiting channel, and then make the first limiting channel in a locked state to constrain the second segment of the first control wire at the first limiting channel. The first segment connected to the second segment can be utilized to manipulate the first movable member of the prosthesis, without the need to first extend the first control wire from the proximal end of the delivery device to the distal end and then extend from the distal end of the delivery device back to the proximal end of the delivery device after passing through the prosthesis, as in the prior art. This avoids the risks of the control wire becoming tangled or jammed, thereby reducing surgical risks.

### BRIEF DESCRIPTION OF THE DRAWINGS

One or more embodiments are illustrated exemplarily with reference to the accompanying drawings, and these exemplary descriptions do not constitute a limitation on the embodiments. Elements having the same reference numeral in the accompanying drawings indicate similar elements. The figures in the accompanying drawings do not constitute a scale limitation unless otherwise stated.
Fig. 1 is a schematic diagram of the coupling between a delivery device at a distal end and a mitral valve clip provided in some embodiments of the present disclosure;
Fig. 2 is a schematic structural diagram of a gripper of the mitral valve clip coupled with the delivery device provided in some embodiments of the present disclosure during movement;
Fig. 3 is a schematic structural diagram of a distal end of the delivery device provided in some embodiments of the present disclosure;
Fig. 4 is a schematic structural diagram of a first control wire provided in some embodiments of the present disclosure;
Fig. 5 is a schematic structural diagram of a distal end of a connecting shaft provided in some embodiments of the present disclosure;
Fig. 6 is a schematic structural diagram of the distal end of the connecting shaft from another perspective, provided in some embodiments of the present disclosure;
Fig. 7 is a schematic structural diagram of the distal end of the connecting shaft from yet another perspective, provided in some embodiments of the present disclosure;
Fig. 8 is a schematic structural diagram of a base of a prosthesis provided in some embodiments of the present disclosure;
Fig. 9 is a schematic structural diagram of the control wire, connecting shaft, and base before they are coupled, provided in some embodiments of the present disclosure;
Fig. 10 is a schematic structural diagram of the control wire, connecting shaft, and base when they are coupled, provided in some embodiments of the present disclosure;
Fig. 11 is a schematic structural diagram of a central rod provided in some embodiments of the present disclosure;
Fig. 12 is a cross-sectional view of a coupling site when the connecting shaft is coupled with the base in the prosthesis system provided in some embodiments of the present disclosure, taken along the axis of the connecting shaft;
Fig. 13 is a cross-sectional view of the connecting shaft and the base provided in some embodiments of the present disclosure, taken along the axis of the connecting shaft;
Fig. 14 is a cross-sectional view of the control wire, the first limiting portion, and the second limiting portion when the connecting shaft is coupled with the base in the prosthesis system provided in some embodiments of the present disclosure, taken along the axis of the connecting shaft;
Fig. 15 is a cross-sectional view of the prosthesis system at the first limiting channel when the connecting shaft is coupled with the base in the prosthesis system provided in some embodiments of the present disclosure;
Fig. 16 is a schematic structural diagram of the coupling process between the connecting shaft and the base in the prosthesis system provided in some embodiments of the present disclosure;
Fig. 17 is a schematic structural diagram of the prosthetic system provided in some embodiments of the present disclosure when the connecting shaft is disengaged from the base;
Fig. 18 is a cross-sectional view of another prosthesis system at the first limiting channel when the connecting shaft is coupled with the base in another prosthesis system provided in some embodiments of the present disclosure;
Fig. 19 is a schematic structural diagram of the mitral valve clip provided in some embodiments of the present disclosure;
Fig. 20 is a schematic structural diagram of the gripper and the control wire when they are engaged, provided in some embodiments of the present disclosure;
Fig. 21 is a schematic structural diagram of another gripper and control wire when they are engaged, provided in some embodiments of the present disclosure; and
Fig. 22 is a schematic structural diagram of yet another clamping arm and control wire when they are engaged, provided in some embodiments of the present disclosure.

### DETAILED DESCRIPTION OF THE EMBODIMENTS

In order to make the objects, technical schemes and advantages of the embodiments of the present disclosure clearer, the embodiments of the present disclosure will be described in detail below with reference to the accompanying drawings. However, those of ordinary skill in the art may understand that in the embodiments of the present disclosure, many technical details are set forth in order to make the reader better understand the present disclosure. However, the technical solutions set forth in the present disclosure may still be implemented even without these technical details and various changes and modifications based on the following embodiments.

In the embodiments of the present disclosure, terms such as "up", "down", "left", "right", "front", "rear", "top", "bottom", "inside", "outside", "middle", "vertical", "horizontal", "transverse", "longitudinal", etc., indicating orientations or positional relationships, are based on the orientations or positional relationships shown in the accompanying drawings. These terms are mainly intended to better describe the present disclosure and its embodiments, and are not intended to limit the indicated devices, elements, or components to have a specific orientation, or to be constructed and operated in a specific orientation.

Moreover, in addition to indicating orientations or positional relationships, the aforementioned terms may also have other meanings. For instance, the term "up" may also be used to indicate a certain attachment or connection relationship in certain situations. For those skilled in the art, the specific meanings of these terms in the present disclosure should be understood based on the specific circumstances.

In addition, the terms "installation", "arrangement", "provided with", "opened", "connection", and "connected" should be understood in a broad sense. For example, the "connection" may be a fixed connection, a detachable connection, or an integral structure; a mechanical connection or an electrical connection; may be a direct connection, an indirect connection through an intermediary, or internal communication between two devices, elements, or components. For those skilled in the art, the specific meanings of these terms in the present disclosure should be understood based on the specific circumstances.

In addition, the terms "first", "second", etc. are mainly used to distinguish different devices, elements, or components (which may or may not have the same specific type and structure), and are not intended to indicate or imply the relative importance and quantity of the indicated devices, elements, or components. The expression "multiple" or "a plurality of" indicates two or more unless otherwise specified.

In the embodiments of the present disclosure, "proximal end" or "proximal side" refers to the end closer to the operator and farther away from the patient. Correspondingly, "distal end" or "distal side" refers to the end closer to the patient and farther away from the operator. In the embodiments of the present disclosure, the terms "parallel" and "vertical" should not be narrowly interpreted as 0° or 90°, but should be understood as reasonable angle ranges including 0° or 90° with some permissible fluctuation, provided that the technical effects are achieved.

Some embodiments of the present disclosure provide a prosthesis system, including: a prosthesis and a delivery device. The prosthesis includes a base and a first movable member. The base has a base body and, a first coupling portion and a first limiting portion arranged on the base body. The delivery device includes a connecting shaft and a first control wire. The connecting shaft includes a shaft body and, a second coupling portion and a second limiting portion arranged on the shaft body. The second coupling portion is configured to detachably couple with the first coupling portion, and the second limiting portion is configured to form a first limiting channel together with the first limiting portion. The first control wire includes a first segment and a second segment connected to each other. The first segment is configured to independently drive the first movable member to move. The first and second limiting portions are configured such that, when the first and second coupling portions are coupled, the first limiting channel in a locked state is formed by the first limiting portion and the second limiting portion, and the second segment is constrained at the first limiting channel; and when the first coupling portion and the second coupling portion are separated, the first limiting channel in an unlocked state is formed by the first limiting portion and the second limiting portion, and the first control wire is detachable from the first limiting channel.

The prosthesis system provided in the embodiments of the present disclosure, enables the delivery device to connect with the prosthesis during minimally invasive surgery via the coupling between the first coupling portion of the base and the second coupling portion of the connecting shaft. The coupling also causes the first limiting portion and the second limiting portion to form the first limiting channel in a locked state, which constrains the second segment of the first control wire, preventing the first control wire from separating from the first limiting channel. As a result, the prosthesis can be delivered to the target position inside the human body through surgical pathways such as human lumens by means of the delivery device. When the prosthesis needs to be manipulated, the first limiting channel remains in the locked state to constrain the second segment of the first control wire at the first limiting channel, so that a portion of the first segment extending out of the first limiting channel can be utilized to adjust the movement of the first movable member of the prosthesis, thereby changing the state of the prosthesis to achieve implantation of the prosthesis, and further achieving the purpose of treatment and diagnosis. After implantation of the prosthesis, the delivery device needs to be disengaged from the prosthesis. This can be achieved by disengaging the first coupling portion from the second coupling portion, so that the first limiting channel is in the unlocked state, and the second segment is no longer constrained by the first limiting channel. As a result, the first control wire can be separated from the first limiting channel, allowing it to be separated from the prosthesis. Furthermore, the base of the prosthesis and the connecting shaft of the delivery device can be detached, so as to detach the delivery device from the prosthesis, thereby facilitating the withdrawal of the delivery device from the human body to complete the implantation surgery. As such, during the implementation of minimally invasive surgery, when it is required to change the state of the prosthesis, the first movable member within the prosthesis can be safely and reliably manipulated through the first control wire of the delivery device, thus changing the state of the prosthesis.

In the embodiments of the present disclosure, when configuring the first control wire and the prosthesis, it is only required to extend the control wire from the proximal end of the delivery device to the distal end of the delivery device and into the first limiting channel, and then make the first limiting channel in a locked state to constrain the second segment of the first control wire at the first limiting channel. The first segment connected to the second segment can be utilized to manipulate the first movable member of the prosthesis, without the need to first extend the first control wire from the proximal end of the delivery device to the distal end and then extend from the distal end of the delivery device back to the proximal end of the delivery device after passing through the prosthesis, as in the prior art. This avoids the risks of the control wire becoming tangled or jammed, thereby reducing surgical risks.

In order to make the objects, technical schemes and advantages of the embodiments of the present disclosure clearer, the embodiments of the present disclosure will be described in detail below with reference to the accompanying drawings.

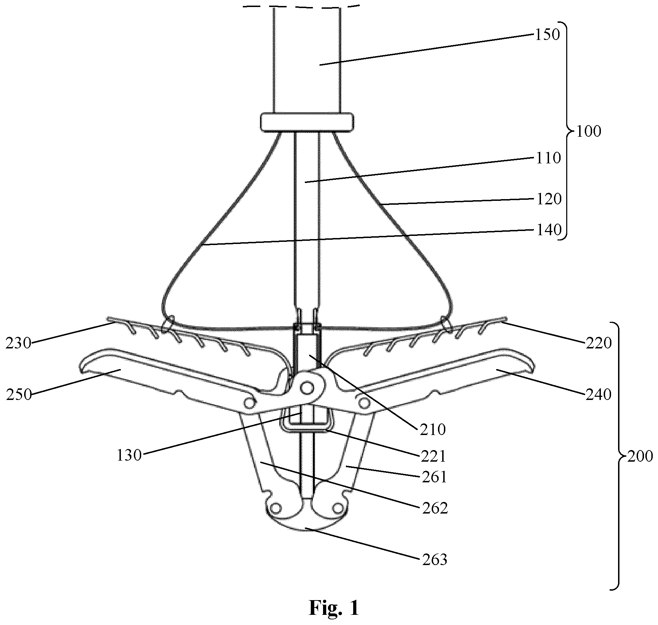

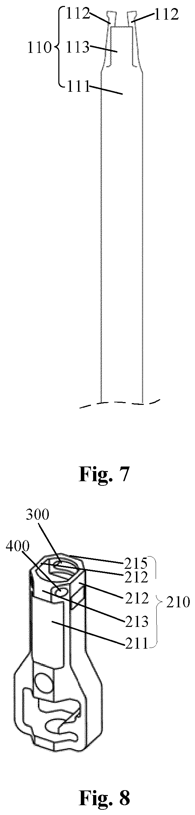

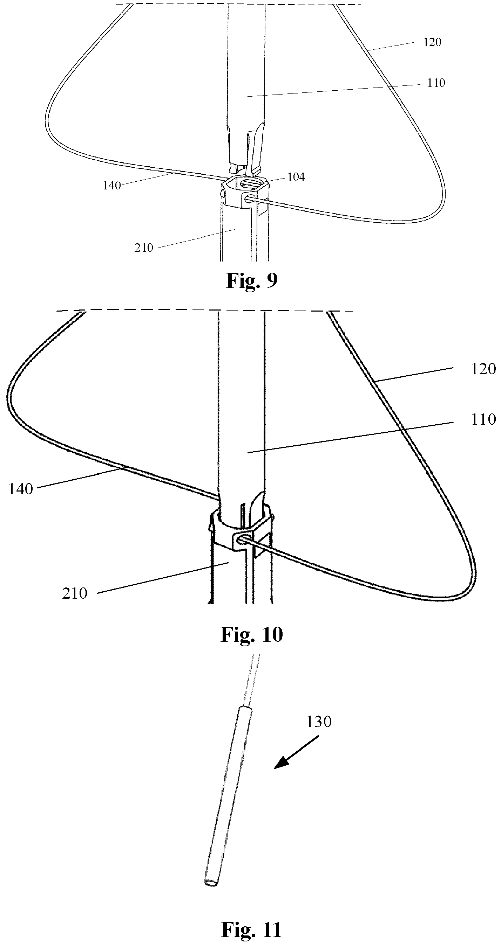

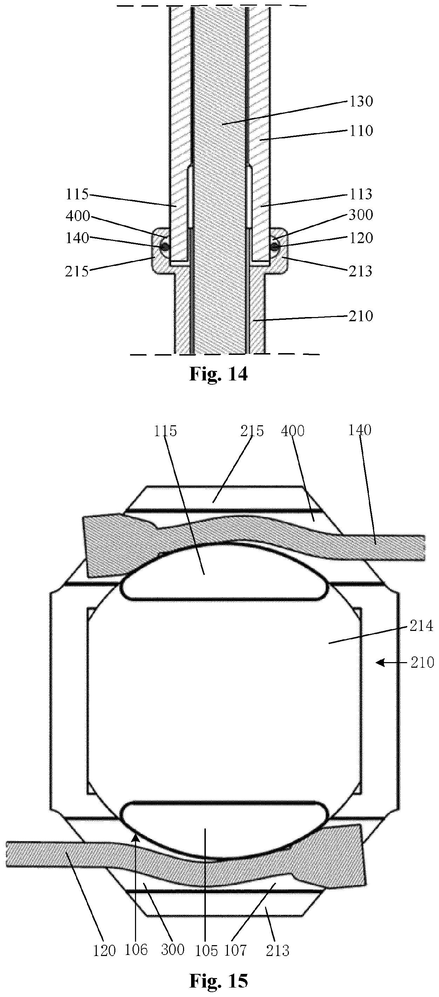

Referring to Figs. 1 to 22, some embodiment of the present disclosure provide a prosthesis system including a prosthesis and a delivery device 100. The prosthesis includes a base 210 and a first movable member. The base 210 has a base body 211 and, a first coupling portion 212 and a first limiting portion 213 arranged on the base body 211. The delivery device 100 includes a connecting shaft 110 and a first control wire 120. The connecting shaft 110 includes a shaft body 111 and, a second coupling portion 112 and a second limiting portion 113 arranged on the shaft body 111. The second coupling portion 112 is configured to detachably couple with the first coupling portion 212, and the second limiting portion 113 is configured to form a first limiting channel 300 together with the first limiting portion 213. The first control wire 120 includes a first segment 121 and a second segment 122 connected to each other. The first segment 121 is configured to independently drive the first movable member to move. The first limiting portion 213 and the second limiting portion 123 are configured such that, when the first coupling portion 212 and the second coupling portion 112 are coupled, the first limiting channel 300 in a locked state is formed by the first limiting portion 213 and the second limiting portion 113, and the second segment 122 is constrained at the first limiting channel 300; and when the first coupling portion 212 and the second coupling portion 112 are separated, the first limiting channel 300 in an unlocked state is formed by the first limiting portion 213 and the second limiting portion 113, and the first control wire 120 is detachable from the first limiting channel 300.

In this way, the arrangement of the first coupling portion 212 and the arrangement of the first limiting portion 213 are independent of each other, simplifying the design and manufacturing of the first coupling portion 212 and the first limiting portion 213. Similarly, the arrangement of the second coupling portion 112 and the arrangement of the second limiting portion 113 are also independent of each other, simplifying the design and manufacturing of the second coupling portion 112 and the second limiting portion 113. Furthermore, when the first coupling portion 212 is coupled with the second coupling portion 112, the first limiting portion 213 and the second limiting portion 113 can cooperate with each other to form the first limiting channel 300 in a locked state.

In some embodiments, the first limiting channel 300 is arranged at an angle to the axis of the connecting shaft 110. Further, an extension direction of the first limiting channel 300 has an angle greater than 0° and less than 180° with the axis of the connecting shaft 110. In an embodiment, the extension direction of the first limiting channel 300 is perpendicular to the axis of the connecting shaft 110.

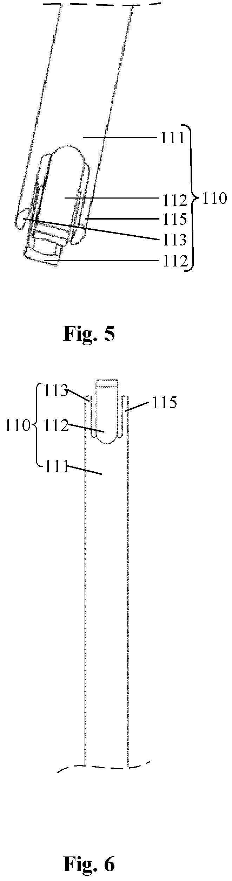

Referring to Figs. 6 to 10, in some embodiments, the first coupling portion 212 and the first limiting portion 213 are arranged at the proximal end of the base body 211, spaced apart along the circumferential direction of the base body 211. The second coupling portion 112 and the second limiting portion 113 are correspondingly arranged at the distal end of the shaft body 111 along the circumferential direction of the shaft body 111.

In some embodiments, the above arrangement refers to that the second coupling portion 112 and the second limiting portion 113 are arranged sequentially along the circumferential direction of the shaft body 111, and the second coupling portion 112 is arranged at a position corresponding to the first coupling portion 212 to form a coupling site, thereby achieving the coupling between the base 210 and the connecting shaft 110; and, the second limiting portion 113 is arranged at a position corresponding to the first limiting portion 213 to form the first limiting channel 300.

In some embodiments, the first coupling portion 212 or the second coupling portion 112 is axially biased relative to the connecting shaft 110; and when the biased coupling portion is driven to be arranged along the axial direction of the connecting shaft 110, the first coupling portion 212 and the second coupling portion 112 are coupled.

It should be noted that, the specific way in which the first coupling portion 212 and the second coupling portion 112 are coupled when the biased coupling portion (the first coupling portion 212 or the second coupling portion 112) is driven to be arranged along the axial direction of the connecting shaft 110 is not limited in the present disclosure.

For example, one of the first coupling portion 212 and the second coupling portion 112 is provided with a protrusion 101, and the other is provided with a groove 102. When the first coupling portion 212 or the second coupling portion 112 is biased from the axial direction of the connecting shaft 110, the protrusion 101 is disengaged from the groove 102. When the biased coupling portion is driven to be arranged along the axial direction of the connecting shaft 110, the protrusion 101 is accommodated in the groove 102. In this way, the protrusion 101 can be accommodated in the groove 102 to achieve coupling between the first coupling portion 212 and the second coupling portion 112, thereby achieving coupling between the connecting shaft 110 and the base 210.

Referring to Figs. 6 to 12, the second coupling portion 112 further includes an inwardly biased support 103. The protrusion 101 is arranged at the distal end of the support 103 and protrudes outwardly perpendicular to the axial direction of the support 103. The base 210 is provided with a receiving channel 104 for receiving the second coupling portion 112, and the groove 102 is arranged on an inner wall surface of the receiving channel 104. When the support 103 is driven to deflect, aligning its axis with the axial direction of the connecting shaft 110, the protrusion 101 is accommodated in the groove 102. In this way, the mechanism enables the function that "when the biased coupling portion is driven to be arranged along the axial direction of the shaft body 111, the protrusion 101 can be accommodated in the groove 102", thereby achieving the connection between the connecting shaft 110 and the base 210.

As an alternative, the second coupling portion 112 further includes an outwardly biased support 103. The protrusion 101 is arranged at the distal end of the support 103 and protrudes inwardly perpendicular to the axial direction of the support 103. The groove 102 is arranged on the outer wall surface of the base 210. When the support 103 is driven to deflect, aligning its axis with the axial direction of the connecting shaft 110, the protrusion 101 is accommodated in the groove 102. In this way, the mechanism enables the function that "when the biased coupling portion is driven to be arranged along the axial direction of the shaft body 111, the protrusion 101 can also be accommodated in the groove 102", thereby achieving the connection between the connecting shaft 110 and the base 210.

It should be noted that in some alternative examples, the support 103 may also be provided with a groove 102, while the base 210 be provided with a protrusion 101 at the position corresponding to the groove 102. In this way, when the support 103 is deflected, the groove 102 on the support 103 can accommodated the protrusion 101, so that the mechanism enables the function that "when the biased coupling portion is driven to be arranged along the axial direction of the shaft body 111, the protrusion 101 can be accommodated in the groove 102", further achieving the connection between the connecting shaft 110 and the base 210.

It should also be noted that in some alternative examples, the support 103 may be arranged on the base 210. The arrangement of the protrusion 101 and the groove 102 can refer to the arrangement described in the aforementioned embodiments, and is not repeated here.

The specific manner for driving the biased coupling portion to be arranged along the axial direction of the connecting shaft 110 is not limited in the present disclosure.

In some embodiments, the delivery device 100 further includes a central rod 130. The central rod 130 is configured such that when a part of the central rod 130 is positioned at the coupling site between the first coupling portion 212 and the second coupling portion 112, the central rod 130 drives the biased coupling portion to be arranged along the axial direction of the connecting shaft 110. Furthermore, when the central rod 130 moves towards the coupling site between the first coupling portion 212 and the second coupling portion 112, the central rod 130 pushes the biased coupling portion, causing the biased coupling portion to deflect towards the axial direction of the connecting shaft 110. When the part of the central rod 130 is positioned at the coupling site between the first coupling portion 212 and the second coupling portion 112, the previously biased coupling portion is now arranged along the axial direction of the connecting shaft 110, thereby facilitating the connection between the connecting shaft 110 and the base 210.

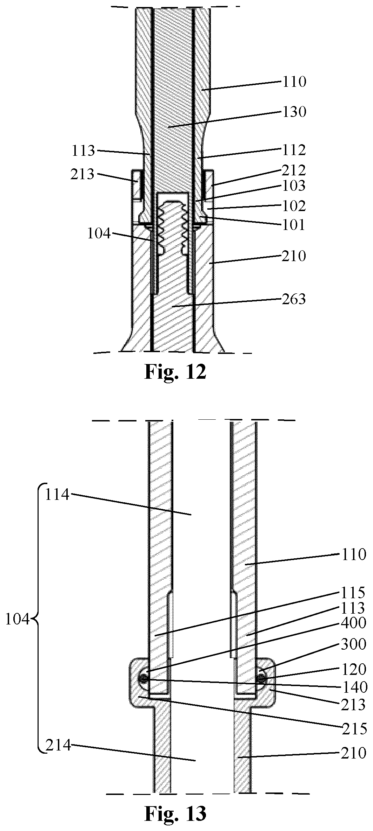

Referring to Figs. 12 to 14, the receiving channel 104 arranged along the axial direction of the connecting shaft 110 may be provided on both the connecting shaft 110 and the base 210, and the central rod 130 is movably arranged in the receiving channel 104. When the central rod 130 is positioned at the coupling site between the connecting shaft 110 and the base 210, the biased coupling portion is driven to be arranged along the axial direction of the connecting shaft 110. In this example, the second coupling portion 112 includes the inwardly biased support 103 mentioned in the above embodiments. After the central rod 130 is positioned into the receiving channel 104, the central rod 130 moves from the proximal end of the connecting shaft 110 towards the distal end. When the central rod 130 passes through the second coupling portion 112 and the first coupling portion 212, the central rod 130 abuts against the support 103, and drives the support 103 to deflect outwardly. When the axis of the support 103 is parallel to the axis of the connecting shaft 110, the protrusion 101 is accommodated in the groove 102, thus achieving the coupling between the connecting shaft 110 and the base 210.

Alternatively, the central rod 130 is a hollow rod. When the central rod 130 is sleeved at the coupling site between the connecting shaft 110 and the base 210, the biased coupling portion is driven to be arranged along the axial direction of the connecting shaft 110. In this example, the second coupling portion 112 includes the outwardly biased support 103 mentioned in the above embodiments. When the central rod 130 moves towards the distal end and sleeves at the coupling site between the second coupling portion 112 and the first coupling portion 212, the central rod 130 abuts against the support 103, causing the support 103 to be driven to deflect inwardly. When the axis of the support 103 is parallel to the axis of the connecting shaft 110, the protrusion 101 is accommodated in the groove 102, thus achieving the coupling between the connecting shaft 110 and the base 210. Furthermore, the central rod 130 may be provided with a relief channel extending axially from the distal end to the proximal end of the central rod 130. The relief channel is arranged to correspond to the first limiting channel 300 along the circumferential direction of the connecting shaft 110, and is configured to allow the first control wire 120 to extend out from the first limiting channel 300, facilitating the first control wire 120 in controlling the first movable member.

The specific manner for forming the first limiting channel 300 by the first limiting portion 213 and the second limiting portion 113 is not limited in the present disclosure.

Referring to Figs. 13 to 15, in some embodiments, one of the first limiting portion 213 and the second limiting portion 113 is provided with a first pin 105, while the other is provided with a first through-slot 107. The first through-slot 107 is arranged at an angle to the connecting shaft 110, and a first opening 106 is provided on a side wall of the first through-slot 107 corresponding to the first pin 105. The first pin 105 can plug into the first through-slot 107 via the first opening 106 to occupy a limited space. When the first coupling portion 212 and the second coupling portion 112 are coupled, the first pin 105 plugs into the first through-slot 107 and occupies the limited space, and the remainder of a space defined by the first through-slot 107 forms the first limiting channel 300 in a locked state. When the first coupling portion 212 and the second coupling portion 112 are separated, the first pin 105 is positioned outside the first through-slot 107, and the space defined by the first through-slot 107 forms the first limiting channel 300 in an unlocked state.

In some embodiments, the radial dimension of the second segment 122 of the first control wire 120 is at least larger than the radial dimension of the portion of the first segment 121 connected to the second segment 122, and the space defined by the first through-slot 107 is configured to accommodate the second segment 122. In this way, the first control wire 120 can move freely within the space defined by the first through-slot 107, and therefore can be detached from the first limiting channel 300. When the first coupling portion 212 and the second coupling portion 112 are coupled, the connecting shaft 110 is secured to the base 210, preventing relative movement between the connecting shaft 110 and the base 210. Simultaneously, the first pin 105 plugs into the first through-slot 107 via the first opening 106. In this way, the first pin 105 occupies a portion of the space defined by the first through-slot 107. Namely, the space occupied by the first pin 105 is the limited space. And the minimum radial dimension of the remainder of the space defined by the first through-slot 107 is configured to be smaller than the radial dimension of the second segment 122, and at least larger than the radial dimension of the portion of the first segment 121 connected to the second segment 122, so that the second segment 122 of the first control wire 120 cannot move freely within the remainder of the space defined by the first through-slot 107 and is constrained in the first limiting channel 300. At this time, the remainder of the space defined by the first through-slot 107 forms the first limiting channel 300 in a locked state.

When the first coupling portion 212 and the second coupling portion 112 are disassembled, the first pin 105 is disengaged from the first through-slot 107. Since the radial dimension of each portion of the space defined by the first through-slot 107 is configured to be equal to or greater than the radial dimension of the second segment 122 to accommodate the second segment 122, the first control wire 120 can move freely within the space defined by the first through-slot 107, and therefore can be detached from the first limiting channel 300. At this time, the space defined by the first through-slot 107 forms the first limiting channel 300 in an unlocked state.

In some embodiments, the first limiting channel 300 in the locked state has a smaller space, while the first limiting channel 300 in the unlocked state has a larger space. By controlling the size of the space of the first limiting channel 300, the first control wire 120 can be constrained or detached.

It should be noted that the arrangement that the first through-slot 107 is arranged at an angle to the connecting shaft 110 can be referred to the aforementioned arrangement that the first limiting channel 300 is arranged at an angle to the connecting shaft 110, and is not further described here. In some embodiments, the extension direction of the first through-slot 107 is perpendicular to the axis of the connecting shaft 110.

The radial dimension in the embodiments refers to the dimension of the cross-section perpendicular to the extension direction. For example, the radial dimension of the first limiting channel 300 refers to the dimension of the cross-section of the first limiting channel 300 perpendicular to its extension direction. In a case that the cross-section of the first limiting channel 300 is circular, the radial dimension of the first limiting channel 300 is the diameter of its cross-section. In a case that the first limiting channel 300 is a non-circular channel, the radial dimension of the first limiting channel 300 is determined according to specific rules. For example, the radial dimension may be defined as twice the distance from the geometric center of its cross-section to a certain point on its cross-section. In some embodiments, the radial dimension of the first limiting channel 300 is defined as the diameter of the circumscribed circle of the cross-section of the first limiting channel 300. In some embodiments, the radial dimension of the first limiting channel 300 is defined as the diameter of the inscribed circle of the cross-section of the first limiting channel 300. In some embodiments, the radial dimension of the first limiting channel 300 is defined as twice the minimum distance among the distances from the geometric center of its cross-section to all points on the edge of the cross-section. In some embodiments, the radial dimension of the first limiting channel 300 is defined as twice the maximum distance among the distances from the geometric center of its cross-section to all points on the edge of the cross-section. A person skilled in the art may select an appropriate rule according to his/her needs. The radial dimensions of various parts of the first control wire 120, as well as those of other components, are also defined in a similar way, and are not further elaborated here.

The embodiments impose no particular limitations on the shape of the second segment 122. For example, the second segment 122 may be linear, spherical, hemispherical, spherical cap-shaped, or ellipsoidal.

In some embodiments, as mentioned above, the receiving channel 104 is provided with the connecting shaft 110, or the receiving channel 104 is provided on the connecting shaft 110 and the base 210. The first through-slot 107 partially intersects with the receiving channel 104 to form an overlapping space, which includes the aforementioned limited space. When the first coupling portion 212 and the second coupling portion 112 are coupled, the first pin 105 plugs into the receiving channel 104 and occupies the limited space within the overlapping space, forming the first limiting channel 300 in the locked state; and when the first coupling portion 212 and the second coupling portion 112 are separated, the first pin 105 is positioned outside the overlapping space, forming the first limiting channel 300 in the unlocked state.

In some embodiments, the receiving channel 104 includes a second receiving section 114 arranged on the connecting shaft 110, and a first receiving section 214 arranged on the base 210. When the first coupling portion 212 and the second coupling portion 112 are coupled, the first pin 105 arranged on the connecting shaft 110 plugs into the first receiving section 214 and occupies the limited space, forming the first limiting channel 300 in the locked state. When the first coupling portion 212 and the second coupling portion 112 are separated, the first pin 105 is positioned outside the overlapping space, forming the first limiting channel 300 in the unlocked state. In an alternative example, when the first coupling portion 212 and the second coupling portion 112 are coupled, the first pin 105 arranged on the base 210 plugs into the second receiving section 114 and occupies the limited space, forming the first limiting channel 300 in the locked state; and when the first coupling portion 212 and the second coupling portion 112 are separated, the first pin 105 is positioned outside the overlapping space, forming the first limiting channel 300 in the unlocked state.

In some embodiments, in a case that the first through-slot 107 is arranged on the second limiting portion 113, the connecting shaft 110 is provided with the receiving channel 104. Further, the receiving channel 104 includes a second receiving section 114 arranged on the connecting shaft 110. When the first coupling portion 212 and the second coupling portion 112 are coupled, the first pin 105 plugs into the second receiving section 114 and occupies the limited space, forming the first limiting channel 300 in the locked state; and when the first coupling portion 212 and the second coupling portion 112 are separated, the first pin 105 is positioned outside the overlapping space, forming the first limiting channel 300 in the unlocked state.

In some embodiments, the first through-slot 107 includes a first end and a second end. The first end corresponds to a side of the second segment 122 away from the first segment 121, and the second end corresponds to a side of the second segment 122 close to the first segment 121. The first end is configured to be closed to prevent the second segment 122 from slipping out. The first segment 121 extends out of the first through-slot through the second end.

It should be noted that the present disclosure does not limit the specific implementation of configuring the first end to be closed. For example, the first end may be closed by using a sealing member to block it. Alternatively, during the formation of the first through-slot, the first end may remain unopened, thereby making the first end closed.

By configuring the first end to be closed, the second segment 122 can be prevented from slipping out the first through-slot through the first end, thereby avoiding the second segment 122 from entanglement or getting stuck with other components or human tissues during surgery, thus reducing surgical risks.

In some alternative embodiments, one of the first limiting portion 213 and the second limiting portion 113 is provided with a second pin, and the other is provided with a second through-slot. The second through-slot is configured such that only the first segment 121 of the first control wire 120 can be accommodated at the radially smallest part of the space defined by the second through-slot, and the second through-slot extends axially towards the second pin and forms a first aperture. The second pin is configured to plug into the second through-slot via the first aperture. When the first coupling portion 212 and the second coupling portion 112 are coupled, the second pin plugs into the first aperture of the second through-slot, and the closed remainder of the space defined by the second through-slot forms the first limiting channel 300 in the locked state. When the first coupling portion 212 and the second coupling portion 112 are separated, the second pin is positioned outside the second through-slot, and the space defined by the second through-slot forms the first limiting channel 300 in the unlocked state.

The present embodiment differs from the above embodiment in that within the space defined by the second through-slot, only the radial dimension of the part of the space away from the second segment 122 is larger than the radial dimension of the first segment 121 of the first control wire 120, while the radial dimensions of the remaining parts of the space are smaller than the radial dimension of the second segment 122 of the first control wire 120, and larger than the radial dimension of at least the part of the first segment 121 connected to the second segment 122. Thus, only part of the space defined by the second through-slot can accommodate the second segment 122 of the first control wire 120, while the remaining parts can only accommodate the first segment 121. Alternatively, the radial dimension of the entire space defined by the second through-slot is smaller than that of the second segment 122 of the first control wire, and larger than the radial dimension of at least the part of the first segment 121 connected to the second segment 122. Thus, the entire space defined by the second through-slot can only accommodate the first segment 121 of the first control wire 120, with the second segment 122 located outside. As a result, the first control wire 120 cannot be directly pulled out. On the other hand, the second through-slot extends axially towards the second pin and forms the first aperture, and the first control wire 120 can be detached from the second through-slot via the first aperture.

Thus, when the first coupling portion 212 and the second coupling portion 112 are coupled, the connecting shaft 110 is secured to the base 210, preventing relative movement between the connecting shaft 110 and the base 210. The second pin plugs into the first aperture of the second through-slot, so the first aperture is blocked by the second pin, and the space defined by the second through-slot is enclosed, forming the first limiting channel 300 in the locked state. The second segment 122 is constrained at the first limiting channel 300, and the first control wire 120 cannot be detached from the second through-slot via the first aperture.

When the first coupling portion 212 and the second coupling portion 112 are separated, and the second pin is disengaged from the first aperture, the space defined by the second through-slot is opened, forming the first limiting channel 300 in the unlocked state. Thus, the first control wire 120 can be detached from the second through-slot via the first aperture.

In the present embodiment, the first limiting channel 300 in the locked state is a closed space, while the first limiting channel 300 in the unlocked state is an opened space. By controlling whether the first limiting channel 300 is closed or not, the first control wire 120 can be constrained or detachable.

In some alternative embodiments, one of the first limiting portion 213 and the second limiting portion 113 is provided with a third pin, and the other is provided with a third through-slot. The third through-slot is configured such that the space defined by the third through-slot can accommodate the first control wire 120, and the third through-slot extends axially towards the third pin and forms a second aperture. The third pin is configured to plug into the third through-slot via the second aperture, to occupy the limited space. The present embodiment differs from the embodiment of the second pin mentioned above in that the plugging of the third pin limits the space formed by the third through-slot, so that the space cannot accommodate the second segment 122 any longer, but still can accommodate the part of the first segment 121 connected to the second segment 122, constraining the second segment 122 at the first limiting channel 300. When the first coupling portion 212 and the second coupling portion 112 are coupled, the third pin plugs into the third through-slot via the second aperture and occupies the limited space, and the remainder of the space defined by the third through-slot forms the first limiting channel 300 in the locked state. When the first coupling portion 212 and the second coupling portion 112 are separated, the third pin is positioned outside the third through-slot, and the space defined by the third through-slot forms the first limiting channel 300 in the unlocked state.

Similar to the embodiment of the first pin mentioned above, the radial dimension of the first limiting channel 300 in the unlocked state is larger than the radial dimension of the second segment 122 of the first control wire 120, so as to accommodate at least the second segment 122 of the first control wire 120 and part of the first segment 121 connected to the second segment 122. The minimum radial dimension of the first limiting channel 300 in the locked state is smaller than the radial dimension of the second segment 122 of the first control wire 120, but larger than the radial dimension of the first segment 121 of the first control wire 120. The present embodiment differs from the above embodiment in that the third pin limits the first limiting channel 300 in the axial direction, so that the second segment 122 is constrained at the first limiting channel 300.

In the present embodiment, the first limiting channel 300 in the locked state has a smaller space, while the first limiting channel 300 in the unlocked state has a larger space. By controlling the size of the space of the first limiting channel 300, the first control wire 120 can be constrained or detached.

In some embodiments, the first coupling portion 212 and the first limiting portion 213 are arranged at the proximal end of the base body 211, spaced apart along the circumferential direction of the base body 211. The second limiting portion 113 is arranged at the distal end of the shaft body 111, and the second coupling portion 112 is arranged on the second limiting portion 113.

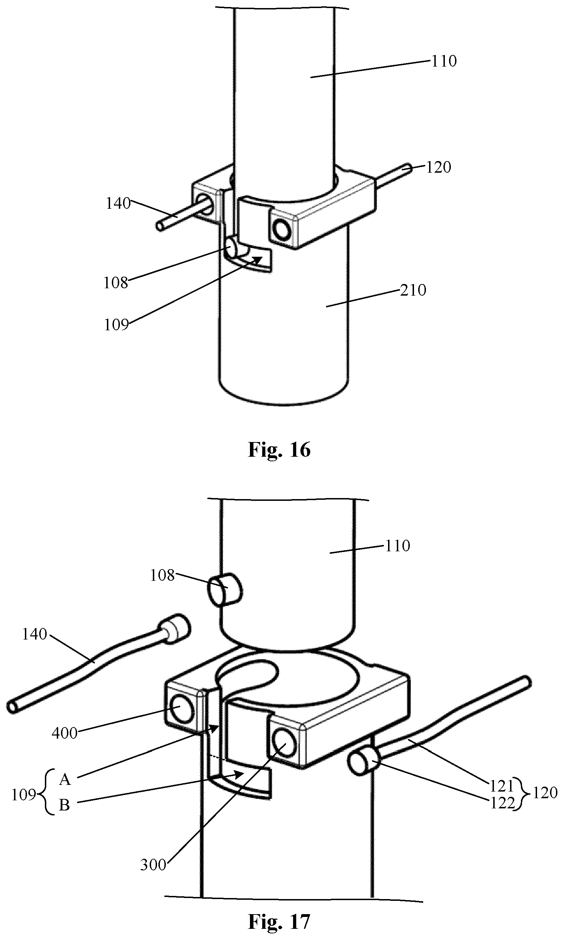

In some embodiments, as shown in Figs. 16 to 18, one of the first coupling portion 212 and the second coupling portion 112 is provided with a protrusion 108, while the other is provided with a bidirectional groove 109 with an open end. The bidirectional groove 109 may extend along the circumferential direction and the axial direction at the same time, or extend along the axial direction first and then along the circumferential direction. The radial dimension of the protrusion 108 matches the radial dimension of the bidirectional groove 109, allowing the protrusion 108 to be accommodated within the bidirectional groove 109. The protrusion 108 enters the bidirectional groove 109 from the open end of the bidirectional groove 109 and moves to the other end of the bidirectional groove 109, achieving the coupling between the first coupling portion 212 and the second coupling portion 112. The protrusion 108 moves from the other end of the bidirectional groove 109 towards the open end of the bidirectional groove 109, until the protrusion 108 disengages from the open end of the bidirectional groove 109, thus achieving the separation of the first coupling portion 212 from the second coupling portion 112.

In some embodiments, the protrusion 108 is arranged at the distal end of the shaft body 111, and the bidirectional groove 109 is arranged at the proximal end of the base body 211. The bidirectional groove 109 includes a first extension portion A and a second extension portion B. The first extension portion A extends axially from the open end towards the distal end, and the second extension portion B extends circumferentially from the other end of the first extension portion A. The first extension portion A and the second extension portion B collectively form an L-shaped configuration. When the first coupling portion 212 and the second coupling portion 112 are required to be coupled, the protrusion 108 enters the first extension portion A from the open end of the bidirectional groove 109 and moves axially towards the distal end, then enters the second extension portion B, and then moves within the second extension portion B to the other end of the bidirectional groove 109, thus achieving the coupling between the first coupling portion 212 and the second coupling portion 112. In the opposite direction, the protrusion 108 moves reversely within the second extension portion B from the other end of the bidirectional groove 109, enters the first extension portion A, and then moves within the first extension portion A toward the open end of the bidirectional groove 109 until it disengages from the open end of the bidirectional groove 109, thereby separating the first coupling portion 212 from the second coupling portion 112. In some embodiments, the inner wall of the base body 211 is provided with an axially extending first shallow groove (not shown in the figures), and the outer wall of the shaft body 111 is provided with an axially extending second shallow groove (not shown in the figures). When the protrusion 108 and the bidirectional groove 109 are coupled, the first shallow groove cooperates with the second shallow groove to form a restriction channel configured to accommodate a restriction wire to further prevent circumferential relative rotation between the connecting shaft 110 and the base 210. Such arrangement makes the coupling between the first coupling portion 212 and the second coupling portion 112 more reliable.

In the present embodiment, the engagement manner between the first limiting portion 213 and the second limiting portion 113 can refer to the above embodiments. However, in the present embodiment, as the first coupling portion 212 requires axial movement relative to the second coupling portion 112 during the coupling process, the second limiting portion 113 can be arranged at the distal end of the shaft body 111, and the second coupling portion 112 can be arranged at the distal end of the shaft body 111 or on the second limiting portion 113, to facilitate the arrangement of the first limiting portion 213. On the other hand, as the first coupling portion 212 requires circumferential movement relative to the second coupling portion 112 during the coupling process, the second limiting portion 113 is a cylindrical pin in some embodiments. In this way, regardless of how the first coupling portion 212 and the second coupling portion 112 rotate, it can be ensured that the first limiting channel in the locked state will not switch to the unlocked state.

In a case that the second limiting portion 113 is a cylindrical first pin 105, when the protrusion 108 enters the bidirectional groove 109, the first pin 105 plugs into the first through-slot 107 via the first opening 106 to occupy the limited space, so that the reminder of the space defined by the first through-slot 107 forms the first limiting channel 300 in the locked state. When the protrusion 108 is positioned outside the bidirectional groove 109, the first pin 105 is positioned outside the first through-slot 107, so that the space defined by the first through-slot 107 forms the first limiting channel 300 in the unlocked state. In a case that the second limiting portion 113 is a cylindrical second or third pin, the corresponding second or third through-slot also has a circular cross-section, and the entrance thereof is provided along the tangential direction to facilitate the entry of the first control wire 120.

It should be noted that in the present embodiment, a third limiting portion 215 may further be provided. The first pin 105 and the third limiting portion 215 engage with each other to form a second limiting channel 400 for working in conjunction with a second control wire 140. The manner that the first pin 105 and the third limiting portion 215 form the second limiting channel 400 can refer to the manner that the first pin 105 and the first limiting portion 213 form the first limiting channel 300, and is not further described here. In this way, one first pin 105 can engage with two limiting portions (i.e., the first limiting portion 213 and the third limiting portion 215).

In addition, the specific manner of detachable coupling between the connecting shaft 110 and the base 210 in the present disclosure is not limited to the embodiments described above. In addition to the manners provided in the aforementioned embodiments, any other suitable manner may also be adopted. For example, the coupling manner shown in Figs. 84 to 88 in the accompanying drawings of the specification of Chinese patent document CN102395331B, as well as the relevant text in the specification.

In some embodiments, the first control wire 120 further includes a third segment, where the third segment and the first segment 121 are arranged on opposite sides of the second segment 122, respectively. When the first limiting channel 300 is in the locked state, the first segment 121 and the third segment cause the second segment 122 to be constrained within the first limiting channel 300; and when the first coupling portion 212 and the second coupling portion 112 are separated, the first limiting channel 300 is in the unlocked state, and the first control wire 120 can be detached from the first limiting channel 300.

For example, the radial dimension of at least the part of the first segment 121 connected to the second segment 122 of the first control wire 120 and the radial dimension of at least the part of the third segment connected to the second segment 122 of the first control wire 120 are both larger than the radial dimension of the second segment 122. One of the first limiting portion 213 and the second limiting portion 113 is provided with the first pin 105, and the other is provided with the first through-slot 107. The radial dimension of the space defined by the first through-slot 107 is configured to be larger than the radial dimension of the third segment and the radial dimension of the first segment 121. In this way, it can be ensured that when the first limiting channel 300 is in the unlocked state, the third segment and the first segment 121 can be pulled out from the first limiting channel 300, facilitating the separation of the first control wire 120 from the prosthesis. On the other hand, the minimum radial dimension of the first limiting channel 300 in the locked state is configured to be smaller than the radial dimension of at least the part of the third segment connected to the second segment 122, smaller than the radial dimension of at least the part of the first segment 121 connected to the second segment 122, and larger than the radial dimension of the second segment 122. Thus, when the first limiting channel 300 is in the locked state, the first segment 121 and the third segment can cause the second segment 122 to be constrained within the first limiting channel 300.

In another example, the radial dimension of at least the part of the first segment 121 connected to the second segment 122 of the first control wire 120 and the radial dimension of at least the part of the third segment connected to the second segment 122 of the first control wire 120 are both larger than the radial dimension of the second segment 122. One of the first limiting portion 213 and the second limiting portion 113 is provided with the second pin, and the other is provided with the second through-slot. The radial dimension of the second through-slot is configured to be smaller than the radial dimension of the first segment 121 and the radial dimension of the third segment, and larger than the radial dimension of the second segment 122. Thus, the second segment 122 is constrained and cannot be pulled out along the axial direction of the first control wire 120, and can be detached only from the first aperture. When the first limiting channel 300 is in the unlocked state, the first aperture is open, so that the second segment 122 can be pulled out from the first aperture of the second through-slot, thereby achieving the separation of the first control wire 120 from the prosthesis. When the first limiting channel 300 is in the locked state, the first aperture is blocked by the second pin, so that the second segment 122 cannot be pulled out from the first aperture and can only be constrained within the first limiting channel 300.

In some embodiments, the prosthesis further includes a second movable member, and the third segment is configured to independently drive the second movable member to move. In this way, using only one control wire (i.e., the first control wire 120) can control the movement of two movable members (i.e., the first movable member and the second movable member).

With further reference to Figs. 1 to 22, the base 210 is further provided with a third limiting portion 215 arranged on the base body 211. The connecting shaft 110 is further provided with a fourth limiting portion 115 arranged on the shaft body 111. The fourth limiting portion 115 is configured to jointly form the second limiting channel 400 with the third limiting portion 215. The prosthesis further includes a second movable member. The delivery device 100 further includes a second control wire 140 having a fourth segment and a fifth segment for independently driving the second movable member to move, where the fourth segment and the fifth segment are connected in sequence. The third limiting portion 215 and the fourth limiting portion 123 are configured such that, when the first coupling portion 212 and the second coupling portion 112 are coupled, the second limiting channel 400 in a locked state is formed by the third limiting portion 215 and the fourth limiting portion 115, and the fourth segment is constrained at the second limiting channel 400; and when the first coupling portion 212 and the second coupling portion 112 are separated, the second limiting channel 400 in an unlocked state is formed by the third limiting portion 215 and the fourth limiting portion 115, and the second control wire 140 is detachable from the second limiting channel 400.

It should be noted that the arrangement relationship between the fourth and fifth segments of the second control wire 140 in the present embodiment can be referred to the arrangement relationship between the first segment 121 and the second segment 122 of the first control wire 120 in the aforementioned embodiment, and is not further elaborated here.

It should also be noted that the arrangement relationship between the second control wire 140 and the second limiting channel 400 formed by the third limiting portion 215 and the fourth limiting portion 115 in the present embodiment can be referred to the arrangement relationship between the first control wire 120 and the first limiting channel 300 formed by the first limiting portion 213 and the second limiting portion 113 in the aforementioned embodiment, and is not further elaborated here.

In addition, the arrangement relationship between the third limiting portion 215 and the fourth limiting portion 115 and the arrangement relationship between the first coupling portion 212 and the second coupling portion 112 in the present embodiment can be referred to the arrangement relationship between the first limiting portion 213 and the second limiting portion 113 and the arrangement relationship between the first coupling portion 212 and the second coupling portion 112 in the aforementioned embodiment, and is not further elaborated here.

In some embodiments, the first limiting portion 213 and the third limiting portion 215 are arranged on opposite sides of the axis of the base body 211, and the positional relationship between the second limiting portion 113 and the fourth limiting portion 115 is set accordingly based on the positional relationship between the first limiting portion 213 and the third limiting portion 215. Such arrangement facilitates the formation of the first limiting portion 213 and the third limiting portion 215 on the base body 21, as well as the formation of the second limiting portion 113 and the fourth limiting portion 115 on the shaft body 111, making it convenient to arrange the coupling portion.

In addition, such configuration allows the first limiting channel 300 formed by the first limiting portion 213 and the second limiting portion 113 and the second limiting channel 400 formed by the third limiting portion 215 and the fourth limiting portion 115 to be arranged oppositely. Consequently, when the first limiting channel 300 constrains the second segment 122 and the second limiting channel 400 constrains the fourth segment, the force acting on the delivery device 100 and even on the prosthesis can be more evenly distributed, while manipulating the first segment 121 of the first control wire 120 and the fifth segment of the second control wire 140.

In some embodiments, the first coupling portion 212 includes multiple first coupling elements (such as protrusions or grooves in the aforementioned embodiment), which are uniformly arranged in the circumferential direction. The second coupling portion 112 includes multiple second coupling elements (such as grooves or protrusions in the aforementioned embodiment), and the arrangement of the second coupling elements corresponds to the arrangement of the first coupling elements. The first coupling elements are designed to detachably couple with the second coupling elements arranged correspondingly.

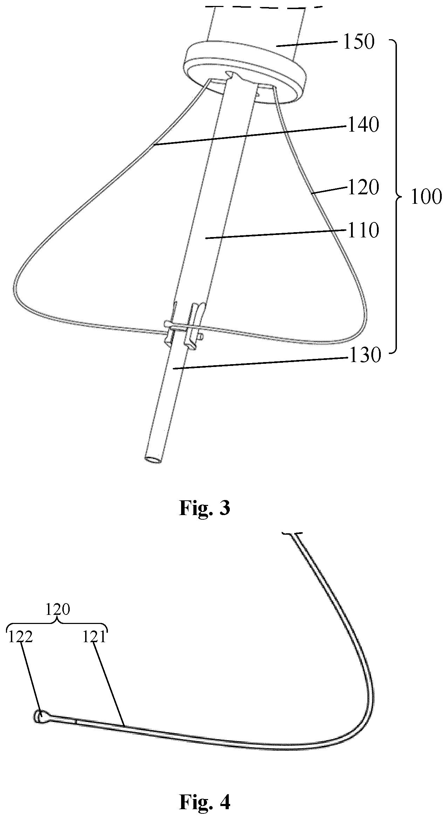

The present disclosure does not impose any limitations on the number and position of the first coupling elements. For example, as shown in Figs. 3, 5, 7, 9, 10, and 12, there are two first coupling elements, which are symmetrically arranged. The first limiting portion 213 is located between two first coupling elements, and the third limiting portion 215 is symmetrically arranged with the first limiting portion 213. In some embodiments, the first limiting portion 213 is located at the angular bisector of the two first coupling elements, meaning that the line connecting the two first coupling elements is perpendicular to the line connecting the first limiting portion 213 and the third limiting portion 215.

The present embodiment does not impose any limitations on the number of movable members in the prosthesis. The prosthesis may include only one movable member, namely the aforementioned first movable member. Controlling the movement of the first movable member via the first segment 121 can achieve changes in the state of the prosthesis.

In some embodiments, the prosthesis may include two movable members, namely a first movable member and a second movable member. Changes of the state of the prosthesis are achieved by controlling the movement of the first movable member via the first segment 121 and controlling the movement of the second movable member via the third segment or the fifth segment. In some embodiments, the prosthesis may include more than two movable members, and the movement of one or more of these movable members can be controlled by the control wire to achieve changes of the state of the prosthesis.

The aforementioned "change of state" may refer to the changes in the structure, size, and shape of the entire or part of prosthesis, such as the transition of the prosthesis from a compressed state to an expanded state. The aforementioned "change of state" may also refer to the implementation of the unlocked state/locked state between the prosthesis and the target tissue, between the prosthesis and the delivery system, and between the internal components of the prosthesis, and/or the mutual transformation between the locked state and the unlocked state. The "change of state" may also refer to changes in the position and/or posture of the entire or part of the prosthesis (such as the first movable member).

Taking a prosthesis including one movable member as an example for explanation, the prosthesis may be a covered stent used to treat aortic stenosis. A restraint wire (i.e., the first movable member) is typically used during delivery to restrain the covered stent in a compressed state. After the covered stent is positioned at the target position, the restraint wire is freed via the control wire, allowing the covered stent to switch from a compressed state to an expanded state. For another example, when delivering the left atrial appendage occluder, the control wire and the left atrial appendage occluder are in a locked state. After the left atrial appendage occluder is released, the control wire is driven to be disengaged from the left atrial appendage occluder, allowing the left atrial appendage occluder to be in an unlocked state.

Taking a prosthesis including two movable members as an example for explanation, the prosthesis may be a valve clip. The valve clip achieves clamping of the valve leaflet edges through the movement of the gripper and the arm to treat valve regurgitation. During the process of clamping the edge of the valve leaflet with the valve clip, the movement of the gripper is controlled via the control wire to change the posture of the gripper.

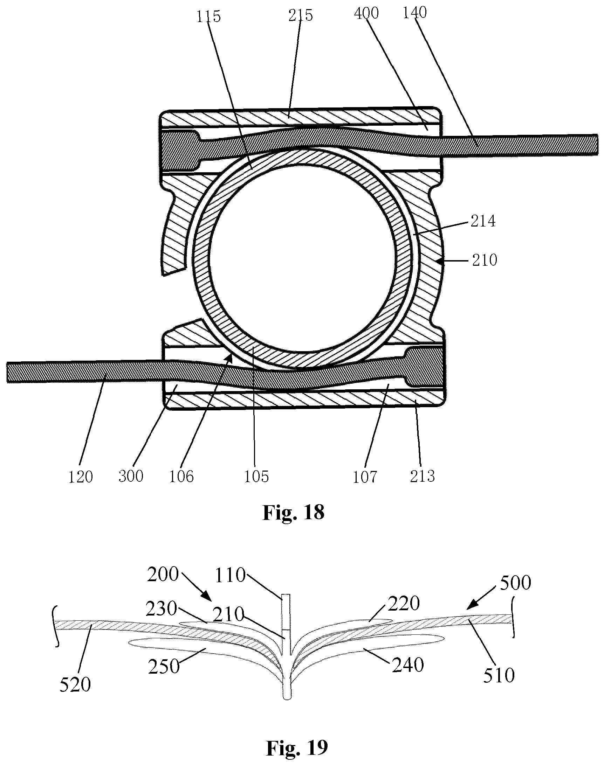

Referring to Fig. 19, a prosthesis, specifically a valve clip for treating mitral regurgitation, namely the mitral valve clip 200, is taken as an example for further explanation. It should be emphasized that the prosthesis being a mitral valve clip 200 is only an example. In some embodiments, the prosthesis may be a valve clip, such as a tricuspid valve clip, used to treat other valve regurgitations. In some embodiments, the prosthesis is an interventional or implantable medical device used for the treatment and diagnosis of other diseases. The present embodiment imposes no limitations on the specific type of prosthesis.

In some embodiments, the mitral valve clip 200 includes a base 210, a gripper (i.e., movable member), and an arm. The gripper and the arm are rotatable relative to the axis of the base 210. The gripper is located at the proximal end of the first base 210, and the arm is located at the distal end of the base 210.

In some embodiments, the gripper includes a first gripper 220 (i.e., first movable member) and a second gripper 230 (i.e., second movable member) which are symmetrically arranged about the axis of the base 210. Each gripper includes a free end and a connecting end. The gripper is rotatable relative to the base 210, allowing the free end to approach or move away from the base.

It still needs to be emphasized that the first movable member here being the first gripper 220 and the second movable member being the second gripper 230 is only an example when the prosthesis is a mitral valve clip 200. In other embodiments, the first movable member may not be the first gripper 220, and the second movable member may not be the second gripper 230. The specific types of the first movable member and the second movable member are not limited in the present disclosure.

Similarly, the arm includes a first arm 240 and a second arm 250, which are symmetrically arranged about the axis of the base 210 and located in the plane defined by the first gripper 220 and the second gripper 230, to better clamp the edges of the anterior leaflet and posterior leaflet of the mitral valve 500. Thus, the first arm 240 and the first gripper 220 form a first clamping area; and the second arm 250 and the second gripper 230 form a second clamping area. Here, the plane defined by the first gripper 220 and the second gripper 230 refers to the plane formed by the axis of the first gripper 220 and the axis of the second gripper 230; the first arm 240 and the second arm 250 being located in the plane defined by the first gripper 220 and the second gripper 230 refers to that the axes of the first arm 240 and the second arm 250 are both located in the plane defined by the first gripper 220 and the second gripper 230. After the mitral valve clip 200 is delivered to the target position of the mitral valve 500, the gripper and the arm are driven to rotate to expand the first clamping area and the second clamping area, so that the edges of the anterior leaflet and posterior leaflets of the mitral valve fall into the clamping areas. Then, the clamping areas are gradually reduced to clamp the anterior leaflet and posterior leaflet of the mitral valve 500 by the gripper and arm, achieving the treatment of mitral regurgitation.

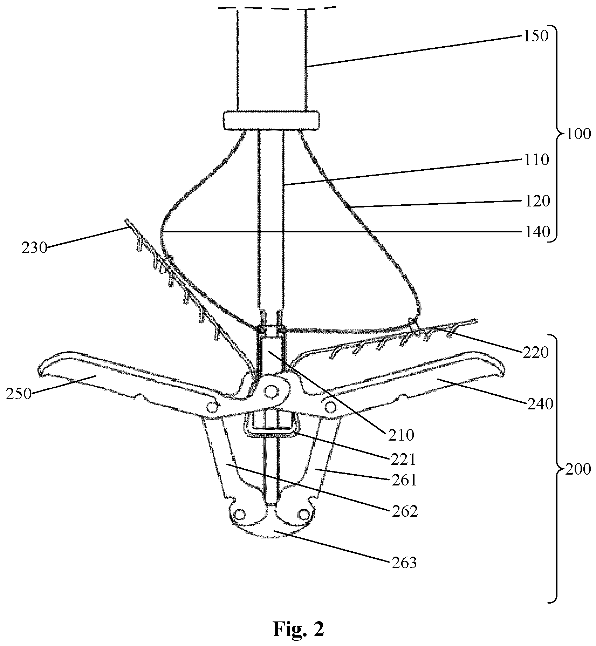

With further reference to Figs. 1 to 3 and Fig. 19, in some embodiments, the first gripper 220 and the second gripper 230 are both in the form of a sheet, and the connecting end of the first gripper 220 is elastically connected to the connecting end of the second gripper 230 via a transition portion 221. The transition portion 221 is arranged at the distal end of the base 210. In some embodiments, the first gripper 220, the transition portion 221, and the second gripper 230 are integrally formed in an inverted omega shape, and the first gripper 220 and the second gripper 230 are inherently in an extended state. When the first segment 121 is pulled, the first gripper 220 is driven to rotate to move upward and closer to the base 210, and the first gripper 220 undergoes elastic deformation. When the first segment 121 is no longer pulled, the first gripper 220 rotates away from the base 210 under its own elastic force, returning to its original state. Similarly, as shown in Fig. 2, when the third/fifth segment is pulled, the second gripper 230 is driven to rotate to move upward and closer to the base 210, and the second gripper 230 undergoes elastic deformation. When the third/fifth segment is no longer pulled, the second gripper 230 rotates away from the base 210, returning to its original state.

With further reference to Figs. 1 to 3 and Fig. 19, the first arm 240 and the second arm 250 are both rotatably connected to the base 210. In some embodiments, the first arm 240 and the second arm 250 are both rotatably connected to the base 210 via a same rotating shaft. In some embodiments, the cross-sectional shape of the first arm 240 perpendicular to the axial direction is U-shaped to better wrap the first leaflet edge 510 of the mitral valve 500 together with the first gripper 220. Similarly, the cross-sectional shape of the second arm 250 perpendicular to the axial direction is U-shaped to better wrap the second leaflet edge 520 of the mitral valve 500 together with the second gripper 230. When the mitral valve clip 200 clamps the leaflets of the mitral valve 500, the first arm 240 and the second arm 250 are driven to rotate, and compress the edges of the anterior leaflet and posterior leaflet of the mitral valve 500 respectively with the first gripper 220 and the second gripper 230, so as to achieve the clamping of the leaflets of the mitral valve 500 by the mitral valve clip 200.

There are no specific limitations on the manner by which the first segment 121 controls the movement of the first movable member, and the manner by which the third/fifth segment controls the movement of the second movable member. For the mitral valve clip 200, it only requires that the first segment 121 is movably connected to the first gripper 220. Thus, on the one hand, the first gripper 220 is rotatable along with the movement of the first segment 121; and on the other hand, when the delivery device 100 is required to be separated from the mitral valve clip 200, the first control wire 120 can be detached from the first gripper 220. The relationship between the third/fifth segment and the second movable member (i.e., the relationship between the third/fifth segment and the second gripper 230) is similar, and is not further elaborated here.

In some embodiments, with further reference to Fig. 1 and Figs. 20 to 22, the first segment 121 extends distally from the first limiting channel 300, and after reaching the first gripper 220, it extends back towards the proximal end of the delivery device 100. In this way, the first segment 121 forms a curved portion at the position near the first gripper 220, and the curved portion interpenetrates with the first gripper 220 or with an attachment arranged on the first gripper 220, so as to control the rotation of the first gripper 220.

In the present embodiment, the second segment 122 is configured to be detachable from the first gripper 220. In this way, when it is required to separate the first control wire 120 from the first gripper 220, it can be ensured that the second segment 122 can be detached from the first gripper 220.

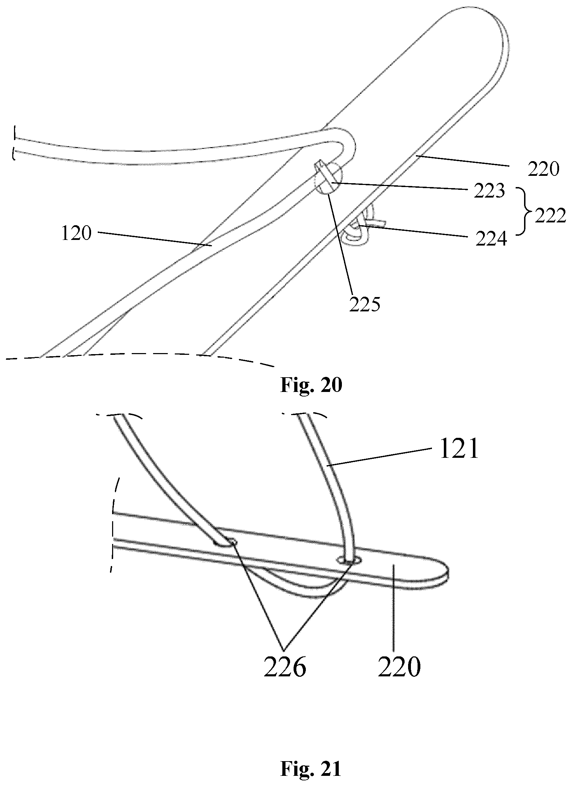

Referring to Fig. 20, in some embodiments, the aforementioned attachment is a fixing wire 222 attached to the first gripper 220 by means of bundling, knotting, or the like, and forms a fixing wire body 223 for interpenetrating with the curved portion of the first segment 121.

In some embodiments, the fixing wire body 223 is annular to form a first through-hole for interpenetrating with the aforementioned curved portion. The fixing wire 222 further includes a fixing knot 224 connected to the fixing wire body 223, and the fixing knot 224 is arranged on the first gripper 220. The curved portion of the first segment 121 passes through the first through-hole to interpenetrate with the fixing wire body 223.

Furthermore, the first gripper 220 is provided with a second through-hole 225 on its surface, and the fixing wire body 223 passes through the second through-hole 225 from the side of the first gripper 220 away from the base 210. The fixing knot 224 is arranged on the side of the first gripper 220 away from the base 210, and the fixing knot 224 is configured to be unable to pass through the second through-hole 225.

Alternatively, the fixing knot 224 of the fixing wire 222 is wound around the second through-hole 225 to be fixedly connected to the first gripper 220.

Referring to Fig. 21, in some embodiments, the first gripper 220 is provided with two third through-holes 226. The end of the first segment 121 away from the second segment 122 passes through one third through-hole 226 from the side of the first gripper 220 away from the first arm 240, i.e., from the side facing away from the first clamping area, and then passes through another third through-hole 226 from the side of the first gripper 220 close to the first arm 240, i.e., the side facing the first clamping area, and then extends in the opposite direction towards the proximal end of the delivery device 100. In this way, the curved portion of the first segment 121 can be formed, allowing the curved portion of the first segment 121 to interpenetrate with the first gripper 220.

Alternatively, the first gripper 220 is provided with an even number of third through-holes 226, more than two in total. The end of the first segment 121 away from the second segment 122 alternatingly passes through these third through-holes 226, and then extends in the opposite direction towards the proximal end of the delivery device 100. In this way, the curved portion of the first segment 121 can also be formed, allowing the curved portion to interpenetrate with the first gripper 220.

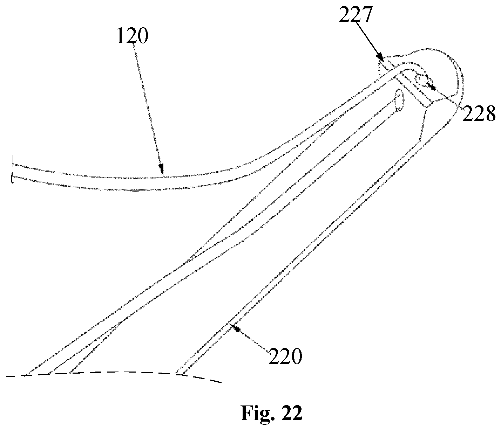

Referring to Fig. 22, in some embodiments, the first gripper 220 is provided with a protrusion 227, and the protrusion 227 on the first gripper 220 is provided with a fourth through-hole 228. The end of the first segment 121 away from the second segment 122 passes through the fourth through-hole 228. In this way, the curved portion of the first segment 121 can also be formed, allowing the curved portion to interpenetrate with the first gripper 220. In some embodiments, an extension direction of the fourth through-hole 228 is the same as an extension direction of the first gripper 220.

It should be noted that the connection manner between the third/fifth segment and the second gripper 230 can refer to the connection manner between the first segment 121 and the first gripper 220 described above, and is not further described here.

In addition, the fourth segment may also be configured to be detachable from the second gripper 230. In this way, when it is required to separate the second control wire 140 from the second gripper 230, it can be ensured that the fourth segment can be detached from the second gripper 230.

In some embodiments, the mitral valve clip 200 further includes: an actuating assembly configured to control the rotation of the first arm 240 and the second arm 250.

With further reference to Figs. 1 and 3, the actuating assembly includes a first connecting rod 261, a second connecting rod 262, and a slider 263. The proximal end of the first connecting rod 261 is rotatably connected to the first arm 240, and the distal end thereof is rotatably connected to the slider 263. The slider 263 can move axially relative to the base 210. Thus, the first connecting rod 261, the first arm 240, the first base 210, and the slider 263 form a kinematic mechanism similar to a crank-slider mechanism. When the slider 263 is driven to move closer or away from the base 210, the first arm 240 is driven to rotate. Similarly, the proximal end of the second connecting rod 262 is rotatably connected to the second arm 250, and the distal end thereof is rotatably connected to the slider 263. Similarly, the second connecting rod 262, the second arm 250, the base 210, and the slider 263 also form a kinematic mechanism similar to the above crank-slider mechanism. When the slider 263 is driven to move closer or away from the base 210, the second arm 250 is driven to rotate.

In some embodiments, the slider 263 is T-shaped, that is, the slider 263 includes a horizontal portion and a vertical portion. The first base 210 is of a hollow structure, and the vertical portion of the slider 263 is movably accommodated in the first base 210. Two ends of the horizontal portion of the slider 263 are rotatably connected to the first connecting rod 261 and the second connecting rod 262 respectively. The vertical portion of the slider 263 is detachably connected to the central rod 130, for example, through a threaded connection. In this way, the central rod 130 can drive the slider 263 to move, to drive the first arm 240 and the second arm 250 to rotate. In some embodiments, the first connecting rod 261 and the second connecting rod 262 are symmetrically arranged about the axis of the base 210. Thus, when the slider 263 drives the first arm 240 and the second arm 250 to move, the rotation angle of the first arm 240 is the same as the rotation angle of the second arm 250.

In some embodiments, the delivery device 100 further includes a catheter 150 sleeved on the connecting shaft 110. The catheter 150 is provided with two channels extending axially along the catheter 150. The end of the first segment 121 away from the second segment 122 passes through one channel from the distal end of this channel and extends to the proximal end of this channel, and the end of the third segment away from the second segment 122 passes through the other channel from the distal end of the other channel and extends to the proximal end of the other channel.

In this way, it is convenient for the operator to pull the end of the first segment 121 away from the second segment 122 at the proximal end of the catheter 150 to adjust the rotation angle of the first gripper 220, and to pull the end of the third/fifth segment away from the fourth segment at the proximal end of the catheter 150 to adjust the rotation angle of the second gripper 230, thereby preventing interference from other components or human tissues.

When it is required to detach the first control wire 120 and the second control wire 140 from the mitral valve clip 200, both the first limiting channel 300 and the second limiting channel 400 are adjusted to the unlocked state, and the operator pulls the end of the first segment 121 away from the second segment 122 at the proximal end of the catheter 150, and pulls the end of the third/fifth segment away from the fourth segment at the proximal end of the catheter 150, thereby achieving withdrawal of the first control wire 120 and the second control wire 140 from the mitral valve clip 200.

The prosthesis system provided in some embodiments of the present disclosure has been introduced in detail above. Specific examples have been illustrated in this paper to elaborate on the principles and embodiments of the present disclosure. The descriptions of the above embodiments are only intended to help understand the present disclosure. There may be changes in specific embodiments and application scope. In summary, the content of this specification should not be construed as a limitation on the present disclosure.

## Claims

1. A prosthesis system, comprising: a prosthesis and a delivery device (100);
wherein the prosthesis comprises a base (210) and a first movable member, the base (210) has a base body (211) and, a first coupling portion (212) and a first limiting portion (213) arranged on the base body (211);
wherein the delivery device (100) comprises a connecting shaft (110) and a first control wire (120), wherein the connecting shaft (110) comprises a shaft body (111) and, a second coupling portion (112) and a second limiting portion (113) arranged on the shaft body (111), wherein the second coupling portion (112) is configured to detachably couple with the first coupling portion (212), and the second limiting portion (113) is configured to form a first limiting channel (300) together with the first limiting portion (213), wherein the first control wire (120) comprises a first segment (121) and a second segment (122) connected to each other, and the first segment (121) is configured to independently drive the first movable member to move;
the first limiting portion (213) and the second limiting portion (123) are configured such that, when the first coupling portion (212) and the second coupling portion (112) are coupled, the first limiting channel (300) in a locked state is formed by the first limiting portion (213) and the second limiting portion (113), and the second segment (122) is constrained at the first limiting channel (300); and when the first coupling portion (212) and the second coupling portion (112) are separated, the first limiting channel (300) in an unlocked state is formed by the first limiting portion (213) and the second limiting portion (113), and the first control wire (120) is detachable from the first limiting channel (300).

2. The prosthesis system according to claim 1, wherein
the first coupling portion (212) and the first limiting portion (213) are arranged at a proximal end of the base body (211), spaced apart along a circumferential direction of the base body (211); and the second coupling portion (112) and the second limiting portion (113) are correspondingly arranged at a distal end of the shaft body (111) along a circumferential direction of the shaft body (111);
or,
the first coupling portion (212) and the first limiting portion (213) are arranged at the proximal end of the base body (211), spaced apart along the circumferential direction of the base body (211); and the second limiting portion (113) is arranged at the distal end of the shaft body (111), and the second coupling portion (112) is arranged on the second limiting portion (113).

3. The prosthesis system according to claim 1, wherein
the first coupling portion (212) or the second coupling portion (112) is axially biased relative to the connecting shaft (110); and
when the biased coupling portion is driven to be arranged along an axial direction of the connecting shaft (110), the first coupling portion (212) and the second coupling portion (112) are coupled.

4. The prosthesis system according to claim 3, wherein
one of the first coupling portion (212) and the second coupling portion (112) is provided with a protrusion (101), and the other is provided with a groove (102);
wherein when the first coupling portion (212) or the second coupling portion (112) is biased from the axial direction of the connecting shaft (110), the protrusion (101) is disengaged from the groove (102); and when the biased coupling portion is driven to be arranged along the axial direction of the connecting shaft (110), the protrusion (101) is accommodated in the groove (102).

5. The prosthesis system according to claim 4, wherein
the second coupling portion (112) further comprises an inwardly biased support (103), wherein the protrusion (101) is arranged at a distal end of the support (103) and protrudes outwardly perpendicular to an axial direction of the support (103); the base (210) is provided with a receiving channel (104) for receiving the second coupling portion (112), and the groove (102) is provided on an inner wall surface of the receiving channel (104); and when the support (103) is driven to deflect so that the support (103) is coaxial with the connecting shaft (110), the protrusion (101) is accommodated in the groove (102);
or,
the second coupling portion (112) further comprises an outwardly biased support (103), wherein the protrusion (101) is arranged at the distal end of the support (103) and protrudes inwardly perpendicular to the axial direction of the support (103); the groove (102) is arranged on an outer wall surface of the base (210); and when the support (103) is driven to deflect so that the support (103) is coaxial with the connecting shaft (110), the protrusion (101) is accommodated in the groove (102).

6. The prosthesis system according to claim 3, wherein
the delivery device (100) further comprises: a central rod (130), wherein the central rod (130) is configured such that when a part of the central rod (130) is positioned at a coupling site between the first coupling portion (212) and the second coupling portion (112), the central rod (130) drives the biased coupling portion to be arranged along the axial direction of the connecting shaft (110).

7. The prosthesis system according to claim 1, wherein
one of the first coupling portion (212) and the second coupling portion (112) is provided with a protrusion (108), while the other is provided with a bidirectional groove (109) with an open end;
wherein the bidirectional groove (109) extends along a circumferential direction and an axial direction at the same time, or extends along the axial direction first and then along the circumferential direction;
a radial dimension of the protrusion (108) matches a radial dimension of the bidirectional groove (109), allowing the protrusion (108) to be accommodated within the bidirectional groove (109);
the protrusion (108) is configured to enter the bidirectional groove (109) from the open end of the bidirectional groove (109) and moves to the other end of the bidirectional groove (109), achieving coupling between the first coupling portion (212) and the second coupling portion (112); and
the protrusion (108) is further configured to move from the other end of the bidirectional groove (109) towards the open end of the bidirectional groove (109), until the protrusion (108) disengages from the open end of the bidirectional groove (109), thus achieving detachment of the first coupling portion (212) from the second coupling portion (112).

8. The prosthesis system according to claim 1, wherein
one of the first limiting portion (213) and the second limiting portion (113) is provided with a first pin (105), while the other is provided with a first through-slot (107), wherein the first through-slot (107) is arranged at an angle to the connecting shaft (110), and a first opening (106) is provided on a side wall of the first through-slot (107) corresponding to the first pin (105), wherein the first pin (105) is configured to plug into the first through-slot (107) via the first opening (106) to occupy a limited space;
when the first coupling portion (212) and the second coupling portion (112) are coupled, the first pin (105) plugs into the first through-slot (107) and occupies the limited space, and the remainder of a space defined by the first through-slot (107) forms the first limiting channel (300) in the locked state; and
when the first coupling portion (212) and the second coupling portion (112) are separated, the first pin (105) is positioned outside the first through-slot (107), and the space defined by the first through-slot (107) forms the first limiting channel (300) in the unlocked state.

9. The prosthesis system according to claim 8, wherein
the receiving channel (104) is provided on the connecting shaft (110), or the receiving channel (104) is provided on the connecting shaft (110) and the base (210);
the first through-slot (107) partially intersects with the receiving channel (104) to form an overlapping space, which comprises the limited space;
when the first coupling portion (212) and the second coupling portion (112) are coupled, the first pin (105) extends into the receiving channel (104) and occupies the limited space within the overlapping space, forming the first limiting channel (300) in the locked state; and
when the first coupling portion (212) and the second coupling portion (112) are separated, the first pin (105) is positioned outside the overlapping space, forming the first limiting channel (300) in the unlocked state.

10. The prosthesis system according to claim 9, wherein
the first through-slot (107) comprises a first end and a second end, wherein the first end is located on a side of the second segment (122) away from the first segment (121), and the second end is located on a side of the second segment (122) close to the first segment (121); the first end is configured to be closed to prevent the second segment (122) from escaping; the first segment (121) extends out of the first through-slot (107) through the second end.

11. The prosthesis system according to any one of claims 8 to 10, wherein
the first pin (105) is cylindrical; the second coupling portion (112) is provided with a protrusion (108), and the first coupling portion (212) is provided with a bidirectional groove (109);
when the protrusion (108) and the bidirectional groove (109) are coupled, the first pin (105) plugs into the first through-slot (107) via the first aperture (106) and occupies the limited space, so that the remainder of the space defined by the first through-slot (107) forms the first limiting channel (300) in the locked state; and
when the protrusion (108) and the bidirectional groove (109) are separated, the first pin (105) is positioned outside the first through-slot (107), so that the space defined by the first through-slot (107) forms the first limiting channel (300) in the unlocked state.

12. The prosthesis system according to claim 1, wherein
one of the first limiting portion (213) and the second limiting portion (113) is provided with a second pin, and the other is provided with a second through-slot, wherein the second through-slot is configured such that only the first segment (121) of the first control wire (120) is receivable at a radially smallest part of a space defined by the second through-slot, and the second through-slot extends axially towards the second pin and forms a first aperture, wherein the second pin is configured to plug into the second through-slot via the first aperture;
when the first coupling portion (212) and the second coupling portion (112) are coupled, the second pin plugs into the first aperture of the second through-slot, and the closed remainder of the space defined by the second through-slot forms the first limiting channel (300) in the locked state; and
when the first coupling portion (212) and the second coupling portion (112) are separated, the second pin is positioned outside the second through-slot, and the space defined by the second through-slot forms the first limiting channel (300) in the unlocked state.

13. The prosthesis system according to claim 12, wherein
the second pin is cylindrical, and the second through-slot has a circular cross-section; the second coupling portion (112) is provided with a protrusion (108), and the first coupling portion (212) is provided with a bidirectional groove (109);
when the protrusion (108) and the bidirectional groove (109) are coupled, the second pin plugs into the second through-slot via the first aperture and occupies the limited space, so that the remainder of the space defined by the second through-slot forms the first limiting channel (300) in the locked state; and
when the protrusion (108) and the bidirectional groove (109) are separated, the second pin is positioned outside the second through-slot, so that the space defined by the second through-slot forms the first limiting channel (300) in the unlocked state.

14. The prosthesis system according to claim 1, wherein
one of the first limiting portion (213) and the second limiting portion (113) is provided with a third pin, and the other is provided with a third through-slot, wherein the third through-slot is configured such that a space defined by the third through-slot is configured to accommodate the first control wire (120), and the third through-slot extends axially towards the third pin and forms a second aperture, wherein the third pin is configured to plug into the third through-slot via the second aperture to occupy the limited space;
when the first coupling portion (212) and the second coupling portion (112) are coupled, the third pin plugs into the third through-slot via the second aperture and occupies the limited space, and the remainder of the space defined by the third through-slot forms the first limiting channel (300) in the locked state; and
when the first coupling portion (212) and the second coupling portion (112) are separated, the third pin is positioned outside the third through-slot, and the space defined by the third through-slot forms the first limiting channel (300) in the unlocked state.

15. The prosthesis system according to claim 14, wherein
the third pin is cylindrical, and the third through-slot has a circular cross-section; the second coupling portion (112) is provided with a protrusion (108), and the first coupling portion (212) is provided with a bidirectional groove (109);
when the protrusion (108) and the bidirectional groove (109) are coupled, the third pin plugs into the third through-slot via the second aperture and occupies the limited space, so that the remainder of the space defined by the third through-slot forms the first limiting channel (300) in the locked state; and
when the protrusion (108) and the bidirectional groove (109) are separated, the third pin is positioned outside the third through-slot, so that the space defined by the third through-slot forms the first limiting channel (300) in the unlocked state.

16. The prosthesis system according to claim 8 or 9, wherein
the first control wire (120) further comprises a third segment, wherein the third segment and the first segment (121) are arranged on opposite sides of the second segment (122), respectively;
when the first limiting channel (300) is in the locked state, the first segment (121) and the third segment cause the second segment (122) to be constrained within the first limiting channel (300); and
when the first coupling portion (212) and the second coupling portion (112) are separated, the first limiting channel (300) is in the unlocked state, and the first control wire (120) is detachable from the first limiting channel (300).

17. The prosthesis system according to claim 16, wherein
a radial dimension of at least the part of the first segment (121) connected to the second segment (122) of the first control wire (120) and a radial dimension of at least the part of the third segment connected to the second segment (122) of the first control wire (120) are both larger than a radial dimension of the second segment (122);
a radial dimension of the space defined by the first through-slot (107) is configured to be larger than the radial dimension of the third segment and the radial dimension of the first segment (121);
a minimum radial dimension of the first limiting channel (300) in the locked state is configured to be smaller than the radial dimension of at least the part of the third segment connected to the second segment (122), smaller than a radial dimension of at least part of the first segment (121) connected to the second segment (122), and larger than the radial dimension of the second segment (122).

18. The prosthesis system according to claim 17, wherein
the prosthesis further comprises a second movable member, and the third segment is configured to independently drive the second movable member to move.

19. The prosthesis system according to claim 12, wherein
the first control wire (120) further comprises a third segment, wherein the third segment and the first segment (121) are arranged on opposite sides of the second segment (122), respectively;
when the first limiting channel (300) is in the locked state, the first segment (121) and the third segment cause the second segment (122) to be constrained within the first limiting channel (300); and
when the first coupling portion (212) and the second coupling portion (112) are separated, the first limiting channel (300) is in the unlocked state, and the first control wire (120) is detachable from the first limiting channel (300).

20. The prosthesis system according to claim 19, wherein
a radial dimension of at least the part of the first segment (121) connected to the second segment (122) of the first control wire (120) and a radial dimension of at least the part of the third segment connected to the second segment (122) of the first control wire (120) are both larger than a radial dimension of the second segment (122);
a radial dimension of the second through-slot is configured to be smaller than the radial dimension of the first segment (121) and the radial dimension of the third segment, and larger than the radial dimension of the second segment (122);
when the first limiting channel (300) is in the unlocked state, the first aperture is open, allowing the second segment (122) to be pulled out from the first aperture of the second through-slot; and
when the first limiting channel (300) is in the locked state, the first aperture is shielded by the second pin to prevent the second segment (122) from being pulled out from the first aperture, and the second segment (122) is constrained within the first limiting channel (300).

21. The prosthesis system according to claim 20, wherein
the prosthesis further comprises a second movable member, and the third segment is configured to independently drive the second movable member to move.

22. The prosthesis system according to claim 14, wherein
the first control wire (120) further comprises a third segment, wherein the third segment and the first segment (121) are located on opposite sides of the second segment (122), respectively;
when the first limiting channel (300) is in the locked state, the first segment (121) and the third segment cause the second segment (122) to be constrained within the first limiting channel (300); and
when the first coupling portion (212) and the second coupling portion (112) are separated, the first limiting channel (300) is in the unlocked state, and the first control wire (120) is detachable from the first limiting channel (300).

23. The prosthesis system according to claim 22, wherein
a radial dimension of at least the part of the first segment (121) connected to the second segment (122) of the first control wire (120) and a radial dimension of at least the part of the third segment connected to the second segment (122) of the first control wire (120) are both larger than a radial dimension of the second segment (122);
a radial dimension of a space defined by the third through-slot is configured to be larger than the radial dimension of the third segment and the radial dimension of the first segment (121);
a minimum radial dimension of the first limiting channel (300) in the locked state is configured to be smaller than the radial dimension of at least the part of the third segment connected to the second segment (122), smaller than a radial dimension of at least part of the first segment (121) connected to the second segment (122), and larger than the radial dimension of the second segment (122).

24. The prosthesis system according to claim 23, wherein
the prosthesis further comprises a second movable member, and the third segment is configured to independently drive the second movable member to move.

25. The prosthesis system according to claim 8, 9, 10, 12 or 14, wherein
the base (210) is further provided with a third limiting portion (215) arranged on the base body (211); the connecting shaft (110) is further provided with a fourth limiting portion (115) arranged on the shaft body (111), wherein the fourth limiting portion (115) is configured to form a second limiting channel (400) together with the third limiting portion (215);
the prosthesis further comprises a second movable member;
the delivery device (100) further comprises a second control wire (140) having a fourth segment and a fifth segment for independently driving the second movable member to move, wherein the fourth segment and the fifth segment are connected in sequence;
the third limiting portion (215) and the fourth limiting portion (123) are configured such that, when the first coupling portion (212) and the second coupling portion (112) are coupled, the second limiting channel (400) in a locked state is formed by the third limiting portion (215) and the fourth limiting portion (115), and the fourth segment is constrained at the second limiting channel (400); and when the first coupling portion (212) and the second coupling portion (112) are separated, the second limiting channel (400) in an unlocked state is formed by the third limiting portion (215) and the fourth limiting portion (115), and the second control wire (140) is detachable from the second limiting channel (400).

26. The prosthesis system according to claim 25, wherein
the first limiting portion (213) and the third limiting portion (215) are arranged on opposite sides of an axis of the base body (211), and a positional relationship between the second limiting portion (113) and the fourth limiting portion (115) is set accordingly based on a positional relationship between the first limiting portion (213) and the third limiting portion (215).

27. The prosthesis system according to claim 25, wherein
there are two first coupling portions (212) symmetrically arranged;
the first limiting portion (213) is located between the two first coupling portions (212), and the third limiting portion (215) is symmetrically arranged with the first limiting portion (213).

28. The prosthesis system according to claim 1, wherein
the prosthesis is configured as a valve clip.

29. The prosthesis system according to claim 28, wherein
the first movable member is configured as a first gripper (220) rotatable about the axis of the base body (211), and the first segment (121) is movably connected to the first gripper (220).
